# EUROPEAN PATENT SPECIFICATION

(11) **EP 2 186 789 B1**
(45) Date of publication and mention of the grant of the patent: **25.12.2013**
(21) Application number: 08827632.4
(22) Date of filing: 20.08.2008
(51) Int. Cl.: C07C 41/26, A61K 8/35, A61K 8/97, A61K 9/48, A61K 31/122, A61K 36/48, A61K 47/10, A61K 47/44, A61K 47/46, C07C 41/46, C07C 43/23

(54) **METHOD OF PRODUCING REDUCED COENZYME Q10 AND METHOD OF STABILIZING THE SAME**
VERFAHREN ZUR HERSTELLUNG VON REDUZIERTEM COENZYM Q10 UND VERFAHREN ZU SEINER STABILISIERUNG
PROCÉDÉ DE PRODUCTION DE LA COENZYME Q10 RÉDUITE ET SON PROCÉDÉ DE STABILISATION

(30) Priority: 22.08.2007 JP 2007216001
(43) Date of publication of application: 19.05.2010
(73) Proprietor: KANEKA CORPORATION, Osaka (JP)
(72) Inventor: UEDA, Takahiro, Osaka (JP); NAGIRA, Yozo, Takasago-shi Hyogo 676-8688 (JP); KITAMURA, Shiro, Takasago-shi Hyogo 676-8688 (JP)
(74) Representative: HOFFMANN EITLE
(86) International application number: PCT/JP2008/064778
(87) International publication number: WO 2009/025277

(56) References cited:
- EP-A1- 1 452 174
- WO-A1-03/084556
- WO-A1-2007/097412
- WO-A1-2007/123044
- WO-A1-2008/084828
- JP-A- 2006 070 016
- JP-A- 2006 328 024
- FUHRMAN B ET AL: "LICORICE EXTRACT AND ITS MAJOR POLYPHENOL GLABRIDIN PROTECT LOW-DENSITY LIPOPROTEIN AGAINST LIPID PEROXIDATION: IN VITRO AND EX VIVO STUDIES IN HUMANS AND IN ATHEROSCLEROTIC APOLIPOPROTEIN E-DEFICIENT MICE", THE AMERICAN JOURNAL OF CLINICAL NUTRITION, AMERICAN SOCIETY FOR NUTRITION, US, vol. 66, no. 2, 1 August 1997 (1997-08-01) , pages 267-275, XP009050255, ISSN: 0002-9165
- PRIOR R L ET AL: "In vivo total antioxidant capacity: comparison of different analytical methods.", FREE RADICAL BIOLOGY & MEDICINE DEC 1999 LNKD- PUBMED:10641708, vol. 27, no. 11-12, December 1999 (1999-12), pages 1173-1181, XP002668709, ISSN: 0891-5849
- WOJCIKOWSKI KEN ET AL: "Antioxidant capacity of 55 medicinal herbs traditionally used to treat the urinary system: a comparison using a sequential three-solvent extraction process", JOURNAL OF ALTERNATIVE AND COMPLEMENTARY MEDICINE, MARY ANN LIEBERT, NEW YORK, NY, US, vol. 13, no. 1, 1 January 2007 (2007-01-01), pages 103-109, XP009123526, ISSN: 1075-5535
- HOLLMAN P C H ET AL: "RELATIVE BIOAVAILABILITY OF THE ANTIOXIDANT FLAVONOID QUERCETIN FROM VARIOUS FOODS IN MAN", FEBS LETTERS, ELSEVIER, AMSTERDAM, NL, vol. 418, 1 January 1997 (1997-01-01), pages 152-156, XP000872805, ISSN: 0014-5793, DOI: 10.1016/S0014-5793(97)01367-7

## Description

### Technical Field

The present invention relates to a production method of reduced coenzyme Q₁₀ using a licorice hydrophobic extract and a stabilization method thereof. As compared to oxidized coenzyme Q₁₀, reduced coenzyme Q₁₀ shows high oral absorbability, and is a compound useful as a superior food, food with nutrient function claims, food for specified health use, nutritional supplement, nutritional product, animal drug, drink, feed, pet food, cosmetic, pharmaceutical product, therapeutic drug, prophylactic drug and the like.

### Background Art

Oxidized coenzyme Q₁₀, which is a benzoquinone derivative known to be widely distributed in the living world, is also called vitamin Q due to its function like a vitamin, and is a component that rejuvenates the body as a nutrient source that brings weak cell activity to a healthy state. On the other hand, reduced coenzyme Q₁₀ is a two-electron reduction form of oxidized coenzyme Q₁₀, and oxidized coenzyme Q₁₀ is an orange crystal, whereas reduced coenzyme Q₁₀ is a white crystal. Reduced coenzyme Q₁₀ and oxidized coenzyme Q₁₀ are localized in mitochondria, lysosome, golgi apparatus, microsome, peroxisome, cellular membrane and the like, and are indispensable substances for the maintenance of biological functions, which are known to be involved in the activation of ATP production, antioxidant action in the body and stabilization of membrane as a constituent component of the electron transport system.

It is known that reduced coenzyme Q₁₀ can be obtained, for example, by a method comprising producing coenzyme Q₁₀ by a conventionally known method such as synthesis, fermentation, extraction from a naturally occurring substance and the like, and concentrating a reduced coenzyme Q₁₀ fraction in an eluate from chromatography and the like (patent reference 1: JP-A-H10-109933). The patent reference 1 describes that, in this case, oxidized coenzyme Q₁₀ contained in the above-mentioned coenzyme Q₁₀ may be reduced with a general reducing agent such as sodium borohydride, sodium hydrosulfite (sodium dithionite) and the like, and concentrated by chromatography, and that the reduced coenzyme Q₁₀ can also be obtained by a method comprising reacting existing highly pure coenzyme Q₁₀ with the above-mentioned reducing agent.

However, reduced coenzyme Q₁₀ obtained according to these methods requires further processing to become usable for edibles, cosmetics and the like.

In addition, reduced coenzyme Q₁₀ is easily oxidized by molecular oxygen into oxidized coenzyme Q₁₀, and therefore, stabilization of reduced coenzyme Q₁₀ is an important issue when it is processed into a food, food with nutrient function claims, food for specified health use, nutritional product, nutritional supplement, animal drug, drink, feed, cosmetic, pharmaceutical product, therapeutic drug, prophylactic drug and the like, or a material or composition therefor, and/or preserved after processing. Complete removal or blocking of oxygen during the above-mentioned processing and preservation is extremely difficult, and remaining or admixed oxygen particularly during heating for processing and long-term preservation exerts a markedly adverse effect. The above-mentioned oxidation is directly related to quality problems such as the by-product oxidized coenzyme Q₁₀.

There are a production method of reduced coenzyme Q₁₀ that can be used as is for edibles and the like, and a method of stably retaining reduced coenzyme Q₁₀ (patent document 2: WO01/52822). The reference describes 1) a composition comprising reduced coenzyme Q₁₀, a reducing agent in an amount effective for eliminating oxidation of reduced coenzyme Q₁₀ into oxidized coenzyme Q₁₀, a surfactant, vegetable oil or a mixture thereof, in an amount effective for dissolving the above-mentioned reduced coenzyme Q₁₀ and the above-mentioned reducing agent, and a solvent as necessary, 2) a composition for oral administration wherein the above-mentioned composition is prepared into a gelatin capsule or a tablet, and 3) a method of preparing the above-mentioned composition containing reduced coenzyme Q₁₀ in situ using oxidized coenzyme Q₁₀ and a reducing agent. The method capable of preparing a reduced coenzyme Q₁₀-containing composition which can be directly used for edibles and the like provides advantages. However, no detailed description relating to the quality, stabilizing effect and the like of the reduced coenzyme Q₁₀ contained in the composition is provided, and the expected level of stabilization is not clear.

In the above-mentioned composition and preparation method thereof, since a wide variety of surfactants are used in large amounts, they are extremely complicated and troublesome. Furthermore, ascorbic acids to be used as reducing agents are oxidized to produce a considerable amount of dehydroascorbic acids, and the dehydroascorbic acids get mixed in with the above-mentioned composition, also posing a problem. Dehydroascorbic acids and oxalic acid produced by decomposition from dehydroascorbic acids are highly noxious, unlike ascorbic acids. For example, an increased amount of lipid peroxide, a decreased amount of antioxidants in the liver and kidney and an increased amount of oxalic acid in the kidney have been reported, and side effects such as decreased resistance to oxidation stress, easy onset of ureteral lithiasis (non-patent reference 1: Nutrition Research Vol. 13, 667-676 (1993)) and the like are feared.

In an attempt to improve the stability of reduced coenzyme Q₁₀ from the above-mentioned problems, a composition containing reduced coenzyme Q₁₀, and fat and oil other than olive oil and/or polyol, and polyglycerin fatty acid ester as necessary, which does not substantially inhibit stabilization of reduced coenzyme Q₁₀ (patent document 3: WO03/62182), and a composition containing reduced coenzyme Q₁₀ and propyleneglycol fatty acid ester in combination (patent document 4: W02007/52802) have also been reported. However, under conditions requiring further oxidization stability, such as long-term preservation, preservation under bad conditions and the like, the oxidization stability of the method sometimes may not be sufficient.

On the other hand, licorice is a plant belonging to the genus Fabaceae Glycyrrhiza widely distributed in the People's Republic of China, Europe, Russian Federation, Republic of Afghanistan, Iran, Islamic Republic of Pakistan and the like. Licorice is used for food additives and foods in Japan. In the US, licorice was registered as a GRAS (Generally Recognized As Safe) food by FDA in 1985, approved as a cancer preventive food in the Designer Foods 1990, and ingestion thereof was recommended. In Europe, licorice is taken daily as licorice sweets such as licorice candy and the like.

In addition, a licorice hydrophobic component extracted from licorice or a licorice water extract residue with an organic solvent and the like has been reported to show many useful actions such as an antioxidant action, an antibacterial action, an enzyme inhibitory action, an antitumor action, an antiallergic action, an antiviral action and the like. The main varieties of licorice include Glycyrrhiza glabra, Glycyrrhiza uralensis (G. uralensis), Glycyrrhiza inflata (G. inflate) and the like. Each variety includes glycyrrhizin (glycyrrhizic acid), which is a hydrophilic component. In addition, licorice polyphenol contained in licorice characteristically contains many prenyl flavonoid, and flavonoid in these hydrophobic components contains a variety-specific compound (non-patent document 2: Progress in the Chemistry of Organic Natural Products, Vol.73, (1998)). The variety-specific flavonoid may also be used for identification of the licorice variety.

Recently, it has been found that, of the extracts obtained from licorice, a licorice hydrophobic extract containing licorice polyphenol and having an ultratrace glycyrrhizin content has a visceral fat reducing action and is useful for the prophylaxis and/or improvement of metabolic syndrome (multiple risk factor syndrome) (patent document 5: WO02/47699). In addition, it has been reported that a licorice hydrophobic extract has a lipase inhibitory action and a cholesterol absorption inhibitory action (patent document 6: W02005/110400), and a component thereof has a PPARγ ligand activity (patent documents 7, 8 and 9: WO03/037316, W02006/085562, WO2007/060992) and the like. In addition, it has been reported that the licorice hydrophobic component has radical eliminating ability (non-patent document 3: The Journal of Alternative and Complementary Medicine, Vol. 13, 103-109 (2007)).

As for licorice, a dry product and a hydrophilic extract having glycyrrhizin as an active ingredient is used solely as a crude drug or in a combination with plural crude drugs. However, a report actively pursuing usefulness of a combination of a licorice hydrophobic extract substantially containing a small amount of glycyrrhizin and other component and the like cannot be found.
patent document 1: JP-A-10-109933
patent document 2: WO01/52822
patent document 3: WO03/62182

Fuhrman et al have reported that polyphenolic flavonoids are powerful antioxidants. In particular, they report that some licorice constituents, one of which was isolated and identified as the isoflavan glabridin, are responsible for its antioxidated characteristics in LDL oxidation (Am J Clin Nutr 1997;66:267-75).

Prior and Cao report on their comparison of different analytical methods to measure in vivo total antioxidant capacity. They conclude that a "battery" of measurements are necessary to adequately access oxidative stress in biological systems and provide a detailed analysis of the different assays which they used in their studies (Free Radical Biology & Medicine, Vol. 27, Nos. 11/12, pp. 1173-1181, 1999).
patent document 4: WO2007/52802
patent document 5: WO02/47699
patent document 6: W02005/110400
patent document 7: WO03/037316
patent document 8: WO2006/085562
patent document 9: WO2007/060992
non-patent document 1: Nutrition Research Vol.13, 667-676 (1993)
non-patent document 2: Progress in the Chemistry of Organic Natural Products, Vol.73, (1998)
non-patent document 3: The Journal of Alternative and Complementary Medicine, Vol.13, 103-109 (2007)

### Disclosure of the Invention

### Problems to be Solved by the Invention

In view of the above, the present invention aims to provide a preferable composition containing reduced coenzyme Q₁₀, which uses a component with past results of use as food and high safety, and is capable of protecting reduced coenzyme Q₁₀ from oxidation and stably retaining the same, when the composition is preserved as it is, or processed into food, food with nutrient function claims, food for specified health uses, nutritional supplement, nutritional supplement, animal drug, beverage, feed, pet food, cosmetic, pharmaceutical product, therapeutic drug, prophylactic drug and the like and preserved, a convenient method thereof, as well as a production method of reduced coenzyme Q₁₀ comprising reducing oxidized coenzyme Q₁₀ to reduced coenzyme Q₁₀.

### Means of Solving the Problems

The present inventors have conducted intensive studies in an attempt to solve the aforementioned problems and first found that a licorice hydrophobic extract has an ability to reduce oxidized coenzyme Q₁₀ to reduced coenzyme Q₁₀. In addition, they have found that a composition containing reduced coenzyme Q₁₀ and a licorice hydrophobic extract in coexistence markedly improves stability of reduced coenzyme Q₁₀.

Hence, the present invention relates to (1) a production method of reduced coenzyme Q₁₀, comprising reducing oxidized coenzyme Q₁₀ to reduced coenzyme Q₁₀ using a licorice hydrophobic extract and (2) a method of stabilizing reduced coenzyme Q₁₀, comprising forming a composition comprising reduced coenzyme Q₁₀ and a licorice hydrophobic extract in coexistence, (3) a composition comprising reduced coenzyme Q₁₀ and a licorice hydrophobic extract having glycyrrhizin content of 0.5 wt% or below and (4) a composition which is in an oral administration form comprising oxidized coenzyme Q₁₀ and licorice hydrophobic extract having a glycyrrhizin content of 0.5 wt% or below.

### Effect of the Invention

The present invention can provide a convenient production method of reduced coenzyme Q₁₀ comprising reducing oxidized coenzyme Q₁₀ to reduced coenzyme Q₁₀ without particularly adding plural components but by simply using a safe licorice hydrophobic extract with past results as food, and a method of stabilizing reduced coenzyme Q₁₀.

Furthermore, the present invention can provide a reduced coenzyme Q₁₀-containing composition, which is stable to oxidation and preferable. The composition of the present invention not only protects reduced coenzyme Q₁₀ in the composition against oxidation and stably retains the same, but also contains a licorice hydrophobic extract useful as a physiologically active component, and therefore, is expected to provide various effects and efficacies as a pharmaceutical product, supplement or food.

### Best Mode for Carrying out the Invention

The present invention is explained in detail in the following. In the present specification, when simply expressed as coenzyme Q₁₀, it includes an oxidized form, a reduced form and a mixture thereof when they are both present.

The present invention relates to (1) a production method of reduced coenzyme Q₁₀, and (2) a stabilization method of reduced coenzyme Q₁₀ and a composition stabilized by the method.

First, a first invention of the present application is explained. The first invention of the present application is directed to a production method of reduced coenzyme Q₁₀ comprising reducing oxidized coenzyme Q₁₀ to reduced coenzyme Q₁₀ using a licorice hydrophobic extract.

In the production method of the first present invention, oxidized coenzyme Q₁₀ to be a starting material may be oxidized coenzyme Q₁₀ alone or a mixture thereof with reduced coenzyme Q₁₀. When the above-mentioned oxidized coenzyme Q₁₀ is a mixture thereof with reduced coenzyme Q₁₀, the ratio of oxidized coenzyme Q₁₀ in the total amount of coenzyme Q₁₀ (i.e., total amount of reduced coenzyme Q₁₀ and oxidized coenzyme Q₁₀) is not particularly limited and is, for example, not less than 1 wt%, normally not less than 5 wt%, preferably not less than 10 wt%, more preferably not less than 20 wt%. The upper limit is not particularly limited, it is normally not more than 99.9 wt%. Needless to say, when oxidized coenzyme Q₁₀ is 100 wt%, the oxidized coenzyme Q₁₀ may be used alone.

The oxidized coenzyme Q₁₀ to be used in the first present invention can be obtained, for example, by a conventionally known method such as synthesis, fermentation, extraction from a naturally occurring substance and the like. Preferably, it is obtained by fermentation or extraction from a naturally occurring substance.

In the present invention, licorice, from which a licorice hydrophobic extract is derived, is not particularly limited as long as it is a plant belonging to the genus licorice (Genus Glycyrrhiza). Specific examples thereof include Glycyrrhiza uralensis (G. uralensis), Glycyrrhiza inflata (G. inflate), Glycyrrhiza glabra (G. glabra), Glycyrrhiza eurycarpa (G. eurycarpa), Glycyrrhiza aspera (G. aspera) and the like. Preferred are G. uralensis, G. inflata, G. glabra and the like, and more preferred is G. glabra.

The licorice hydrophobic extract to be used in the present invention is an extract of licorice mainly containing an oily (hydrophobic) component of licorice. The method for obtaining the licorice hydrophobic extract is not particularly limited, and known methods can be used. For example, it can be obtained by extracting the above-mentioned licorice or a powder thereof, or a water extract residue thereof (e.g., a licotice residue after extracting or removing hydrophilic component in licorice with water or aqueous alkaline solution, or the residue after drying) with an organic solvent, fat, oil or a mixture of fat and oil. Alternatively, it can be obtained by further extracting a hydrophobic extract once extracted by the above-mentioned method with a different kind of organic solvent, fat, oil or a mixture of fat and oil. As the extraction solvent to be used herein, those approved for use for the production and processing of pharmaceutical products, foods, food additives and the like are preferable, and examples thereof include alcohols, fatty acid esters, ketones, hydrocarbons, fat, oil and a mixture of fat and oil, and the like to be mentioned below. Needless to say, these extraction solvents may be used alone, or as a mixed solvent of at least two kinds thereof.

Among the above-mentioned extraction solvents, ethanol, ethyl acetate, acetone, hexane, heptane, fat, oil and a mixture of fat and oil and the like are preferably used. As the fat, oil and a mixture of fat and oil, medium-chain triglyceride (MCT) and partial glycerides of fatty acid are preferably used, and a combination of medium-chain triglyceride and ethanol and the like is particularly preferably used, from the aspects of extraction rate and the like. As the above-mentioned extraction solvent, a water-containing solvent may be used. However, the water content of the extraction solvent is preferably low so as to suppress the glycyrrhizin content of licorice hydrophobic extract to allow level for the below-mentioned purpose.

An extract obtained by extracting a hydrophobic component of licorice, or the extract after removal of an extraction solvent is used as a licorice hydrophobic extract in the present invention. In the present invention, the licorice hydrophobic extract may be a direct extract, or may be further crudely purified or purified by a purification step such as column treatment, deodorization treatment, decolorization treatment and the like before use.

The oily (hydrophobic) component to be mainly contained in the licorice hydrophobic extract to be used in the present invention contains licorice polyphenol. The licorice hydrophobic extract to be used in the present invention preferably contains licorice polyphenol in a large amount. From this aspect, the content of licorice polyphenol contained in the licorice hydrophobic extract to be used in the present invention is preferably not less than 50 wt%, more preferably not less than 60 wt%, in dry weight ratio.

The licorice polyphenol to be contained in the licorice hydrophobic extract of the present invention is not particularly limited as long as it is a polyphenol component contained in the above-mentioned licorice. Specific examples thereof include glycycoumarin, glycyrol, glycyrin, liquiritigenin, glicoricone, glabridin, glabrene, glabrol, 3'-hydroxy-4'-O-methylglabridin, 4'-O-methylglabridin, glyurallin B, licocoumarone, gancaonin I, dehydroglyasperin D, echinatin, isolicoflavonol, dehydroglyasperin C, glyasperin B, glycyrrhisoflavanone, lupiwighteone, glyasperin D, semilicoisoflavone B and the like.

The licorice hydrophobic extract of the present invention contains at least one kind of licorice polyphenol from among such licorice polyphenol components. Among these, the composition of the present invention preferably contains at least any of glabridin, glabrene, glabrol, 3'-hydroxy-4'-O-methylglabridin and 4'-O-methylglabridin, and more preferably contains at least glabridin.

Polyphenol is a compound having a structure with plural phenol groups, and its general interpretation includes any compound wherein plural hydroxyl groups are bonded to benzene ring. The amount of polyphenol can be measured by, for example, colorimetric methods such as iron tartrate method, Persian blue method, Folin-Ciocalteu assay, Folin-Denis assay and the like, measurement per components by HPLC and the like, and any measurement method can be used. For the measurement of the whole amount of polyphenol, Folin-Denis assay or Folin-Ciocalteu assay is often used. In the case of Folin-Denis assay or Folin-Ciocalteu assay, a standard substance is used to draw an analytical curve of the standard substance, and the whole amount of polyphenol is determined by conversion based on the standard substance.

In the case of a licorice hydrophobic extract, G. glabra, for example, the glabridin can be used as the standard substance. To be specific, for example, the content of the licorice polyphenol component in the licorice hydrophobic extract to be used can be determined on the basis of glabridin by the method described in the below-mentioned Examples.

In the present invention, moreover, the above-mentioned purified product of licorice polyphenol itself (e.g., those isolated and purified, reagent, chemically synthesized product, etc.) can also be used as the licorice hydrophobic extract.

In addition, in the licorice hydrophobic extract to be used in the present invention, components other than licorice polyphenol, for example, glycyrrhizin, which is a hydrophilic component, may be contained depending on the extraction conditions. In the present invention, the content of glycyrrhizin in the licorice hydrophobic extract to be used is preferably small, and a licorice hydrophobic extract wherein at least the glycyrrhizin content is preferably not more than the content of a licorice polyphenol component (e.g., glabridin) is preferably used. Specifically, use of an extract having a glycyrrhizin content of 0.5 wt% or below is preferable, use of an extract having a glycyrrhizin content of 0.1 wt% or below is further preferable, use of an extract having a glycyrrhizin content of 0.05 wt% or below is particularly preferable, and use of an extract substantially free of glycyrrhizin (e.g., below detection limit) is most preferable.

In the first (production method) of the present invention, the concentration of the licorice hydrophobic extract to the reaction system (total weight of all reaction mixtures) at the time when the reaction is started is not particularly limited. However, from the aspect of efficient reduction of oxidized coenzyme Q₁₀ to reduced coenzyme Q₁₀, it is normally not less than about 1 wt%, preferably not less than about 3 wt%, more preferably not less than about 5 wt%, particularly preferably not less than about 10 wt%, based on the dry weight of the extract. While the upper limit is not particularly limited, it is normally not more than about 70 wt%, preferably not more than about 60 wt%, more preferably not more than about 50 wt%, so as to ensure appropriate properties and the like.

In the first (production method) of the present invention, the concentration of oxidized coenzyme Q₁₀ to the reaction system (total weight of all reaction mixtures) at the time when the reaction is started is not particularly limited. It is normally not less than about 0.01 wt%, preferably not less than about 0.05 wt%, more preferably not less than about 0.1 wt%, particularly preferably not less than about 0.2 wt%, further preferably not less than about 0.5 wt%. Among these, it is not less than about 1 wt%. While the upper limit is not particularly limited, it is normally not more than 90 wt%, preferably not more than 60 wt%, more preferably not more than 50 wt%, still more preferably not more than 30 wt%, so as to ensure solubility of oxidized coenzyme Q₁₀ in the reaction system and the necessary amount of the licorice hydrophobic extract which is a reducing agent.

In the first (production method) of the present invention, the weight ratio of oxidized coenzyme Q₁₀ and licorice hydrophobic extract at the time when the reaction is started is not particularly limited. The weight ratio of oxidized coenzyme Q₁₀ to the licorice hydrophobic extract (oxidized coenzyme Q₁₀ weight/licorice hydrophobic extract weight) is normally not less than about 1/99, preferably not less than about 5/95, more preferably not less than about 10/90, particularly preferably not less than about 20/80. While the upper limit of the weight ratio of oxidized coenzyme Q₁₀ to the licorice hydrophobic extract is not particularly limited, it is, for example, not more than about 90/1, preferably not more than about 80/20, more preferably not more than about 70/30, particularly preferably not more than about 60/40.

In the first (production method) of the present invention, oxidized coenzyme Q₁₀ and licorice hydrophobic extract, which are the starting materials, only need to be in contact with each other in the reaction system, where the system is not particularly limited and may be homogenous or nonhomogenous. For example, it may be a reaction system wherein oxidized coenzyme Q₁₀ and a licorice hydrophobic extract are in contact with each other as a solid, wherein one is present in a liquid layer or dissolved in a solvent and the like, and the other is present as a solid in the liquid layer, wherein oxidized coenzyme Q₁₀ is present as a melt, and a licorice hydrophobic extract is present as a solid in the melt, wherein both are present in the liquid layer, forming liquid-liquid two layers, wherein both are present in the same liquid phase and the like. Needless to say, a system showing high contact efficiency between oxidized coenzyme Q₁₀ and a licorice hydrophobic extract is effective for the reduction of oxidized coenzyme Q₁₀. From this aspect, most preferred is the coexistence of oxidized coenzyme Q₁₀ and licorice hydrophobic extract in the same liquid phase. In addition, a composition containing oxidized coenzyme Q₁₀ and a licorice hydrophobic extract, such as the above-mentioned reaction system, is also encompassed in the present invention.

From the above aspects, when performing the reduction reaction in the first of the present invention, a solvent is preferably used to allow existence of oxidized coenzyme Q₁₀ and/or licorice hydrophobic extract in the liquid phase. The solvent to be used for the reduction reaction in the first of the present invention is not particularly limited, and examples thereof include organic solvents such as hydrocarbons, fatty acid esters, ethers, alcohols, ketones, fatty acids, nitrogen compounds (including nitriles and amides), sulfur compounds and the like, fat, oil and a mixture of fat and oil, water and the like. Since oxidized coenzyme Q₁₀ and licorice hydrophobic extract are liposoluble, when water is used as a solvent, it is preferably used as a mixture thereof with other solvent.

While the above-mentioned hydrocarbons are not particularly limited, for example, aliphatic hydrocarbon, aromatic hydrocarbon, halogenated hydrocarbon and the like can be recited. Particularly, aliphatic hydrocarbon and aromatic hydrocarbon are preferable, and aliphatic hydrocarbon is especially preferable.

While aliphatic hydrocarbon may be cyclic or non-cyclic, saturated or unsaturated and is not particularly limited, a non-cyclic aliphatic hydrocarbon is particularly preferably used. Generally, aliphatic hydrocarbon having 3 to 20 carbon atoms, preferably 5 to 12 carbon atoms, can be used.

Specific examples include propane, butane, isobutane, pentane, 2-methylbutane, cyclopentane, 2-pentene, hexane, 2-methylpentane, 2,2-dimethylbutane, 2,3-dimethylbutane, methylcyclopentane, cyclohexane, 1-hexene, cyclohexene, heptane, 2-methylhexane, 3-methylhexane, 2, 3-dimethylpentane, 2,4-dimethylpentane, methylcyclohexane, 1-heptene, octane, 2,2,3-trimethylpentane, isooctane, ethylcyclohexane, 1-octene, nonane, 2,2,5-trimethylhexane, 1-nonene, decane, 1-decene, p-menthane, undecane, dodecane and the like.

Among these, saturated aliphatic hydrocarbon having 5 to 8 carbon atoms is preferable, and pentane, 2-methylbutane, cyclopentane, hexane, 2-methylpentane, 2,2-dimethylbutane, 2,3-dimethylbutane, methylcyclopentane, cyclohexane, heptane, 2-methylhexane, 3-methylhexane, 2,3-dimethylpentane, 2,4-dimethylpentane, methylcyclohexane, octane, 2,2,3-trimethylpentane, isooctane, ethylcyclohexane and the like are particularly preferable.

While aromatic hydrocarbon is not particularly limited, normally, aromatic hydrocarbon having 6 to 20 carbon atoms, particularly 6 to 12 carbon atoms, especially 7 to 10 carbon atoms, is preferably used. Specific examples include benzene, toluene, xylene, o-xylene, m-xylene, p-xylene, ethylbenzene, cumene, mesitylene, tetralin, butylbenzene, p-cymene, cyclohexylbenzene, diethylbenzene, pentylbenzene, dipentylbenzene, dodecylbenzene, styrene and the like. It is preferably toluene, xylene, o-xylene, m-xylene, p-xylene, ethylbenzene, cumene, mesitylene, tetralin, butylbenzene, p-cymene, cyclohexylbenzene, diethylbenzene or pentylbenzene, more preferably, toluene, xylene, o-xylene, m-xylene, p-xylene, cumene or tetralin, and most preferably cumene.

Halogenated hydrocarbon may be cyclic or non-cyclic, saturated or unsaturated, and is not particularly limited. In general, non-cyclic one is preferably used. Normally, chlorinated hydrocarbon and fluorinated hydrocarbon are preferable, and chlorinated hydrocarbon is particularly preferable. A halogenated hydrocarbon having 1 to 6 carbon atoms, particularly 1 to 4 carbon atoms, especially 1 or 2 carbon atoms, is preferably used.

Specific examples include dichloromethane, chloroform, carbon tetrachloride, 1,1-dichloroethane, 1,2-dichloroethane, 1,1,1-trichloroethane, 1,1,2-trichloroethane, 1,1,1,2-tetrachloroethane, 1,1,2,2-tetrachloroethane, pentachloroethane, hexachloroethane, 1,1-dichloroethylene, 1,2-dichloroethylene, trichloroethylene, tetrachloroethylene, 1,2-dichloropropane, 1,2,3-trichloropropane, chlorobenzene, 1,1,1,2-tetrafluoroethane and the like.

It is preferably dichloromethane, chloroform, carbon tetrachloride, 1,1-dichloroethane, 1,2-dichloroethane, 1,1,1-trichloroethane, 1,1,2-trichloroethane, 1,1-dichloroethylene, 1,2-dichloroethylene, trichloroethylene, chlorobenzene or 1,1,1,2-tetrafluoroethane, more preferably dichloromethane, chloroform, 1,2-dichloroethylene, trichloroethylene, chlorobenzene or 1,1,1,2-tetrafluoroethane.

While fatty acid esters are not particularly limited, for example, propionate, acetate, formate and the like can be used. Particularly, acetate and formate are preferable, and acetate is especially preferable. While ester group is not particularly limited, in general, alkyl ester or aralkyl ester having 1 to 8 carbon atoms, preferably alkyl ester having 1 to 6 carbon atoms, more preferably alkyl ester having 1 to 4 carbon atoms, is preferably used.

Examples of propionate include methyl propionate, ethyl propionate, butyl propionate and isopentyl propionate.

Examples of acetate include methyl acetate, ethyl acetate, propyl acetate, isopropyl acetate, butyl acetate, isobutyl acetate, sec-butyl acetate, pentyl acetate, isopentyl acetate, sec-hexyl acetate, cyclohexyl acetate, benzyl acetate and the like. It is preferably methyl acetate, ethyl acetate, propyl acetate, isopropyl acetate, butyl acetate, isobutyl acetate, sec-butyl acetate, pentyl acetate, isopentyl acetate, sec-hexyl acetate or cyclohexyl acetate, more preferably methyl acetate, ethyl acetate, propyl acetate, isopropyl acetate, butyl acetate or isobutyl acetate, and most preferably ethyl acetate.

Examples of formate include methyl formate, ethyl formate, propyl formate, isopropyl formate, butyl formate, isobutyl formate, sec-butyl formate, pentyl formate and the like. It is preferably methyl formate, ethyl formate, propyl formate, butyl formate, isobutyl formate or pentyl formate, and most preferably ethyl formate.

The above-mentioned ethers may be cyclic or non-cyclic, saturated or unsaturated, and are not particularly limited. Generally, saturated ones are preferably used. Normally, ether having 3 to 20 carbon atoms, particularly 4 to 12 carbon atoms, especially 4 to 8 carbon atoms, is preferably used.

Specific examples include diethyl ether, methyl tert-butyl ether, dipropyl ether, diisopropyl ether, dibutyl ether, dihexyl ether, ethylvinyl ether, butylvinyl ether, anisole, phenetole, butylphenyl ether, methoxytoluene, dioxane, furan, 2-methylfuran, tetrahydrofuran, tetrahydropyran, ethylene glycol dimethyl ether, ethylene glycol diethyl ether, ethylene glycol dibutyl ether, ethylene glycol monomethyl ether, ethylene glycol monoethyl ether, ethylene glycol dibutyl ether and the like.

Preferred are diethyl ether, methyl tert-butyl ether, dipropyl ether, diisopropyl ether, dibutyl ether, dihexyl ether, ethyl vinyl ether, butyl vinyl ether, anisole, phenetole, butylphenyl ether, methoxytoluene, dioxane, 2-methylfuran, tetrahydrofuran, tetrahydropyran, ethylene glycol dimethyl ether, ethylene glycol diethyl ether, ethylene glycol dibutyl ether, ethylene glycol monomethyl ether and ethylene glycol monoethyl ether, more preferred are diethyl ether, methyl tert-butyl ether, anisole, dioxane, tetrahydrofuran, ethylene glycol monomethyl ether, ethylene glycol monoethyl ether, more preferably, diethyl ether, methyl tert-butyl ether, anisole and the like, and most preferred is methyl tert-butyl ether.

The above-mentioned alcohols may be cyclic or non-cyclic, saturated or unsaturated, and are not particularly limited, and generally, saturated ones are preferably used. Normally, a monovalent alcohol having 1 to 20 carbon atoms, particularly 1 to 12 carbon atoms, especially 1 to 6 carbon atoms, among others 1 to 5 carbon atoms, is preferable, a divalent alcohol having 2 to 5 carbon atoms is preferable, and a trivalent alcohol having 3 carbon atoms is preferable.

Examples of the monovalent alcohol include methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol, neopentyl alcohol, 1-hexanol, 2-methyl-1-pentanol, 4-methyl-2-pentanol, 2-ethyl-1-butanol, 1-heptanol, 2-heptanol, 3-heptanol, 1-octanol, 2-octanol, 2-ethyl-1-hexanol, 1-nonanol, 1-decanol, 1-undecanol, 1-dodecanol, allyl alcohol, propargyl alcohol, benzyl alcohol, cyclohexanol, 1-methylcyclohexanol, 2-methylcyclohexanol, 3-methylcyclohexanol, 4-methylcyclohexanol and the like.

Preferred are methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol, - neopentyl alcohol, 1-hexanol, 2-methyl-1-pentanol, 4-methyl-2-pentanol, 2-ethyl-1-butanol and cyclohexanol, more preferred are methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol, neopentyl alcohol, further preferably, methanol, ethanol, 1-propanol, 2-propanol, 1-butanol, 2-butanol, isobutyl alcohol, 2-methyl-1-butanol, isopentyl alcohol, and most preferred is ethanol.

Examples of the divalent alcohol include 1,2-ethanediol, 1,2-propanediol, 1,3-propanediol, 1,2-butanediol, 1,3-butanediol, 1,4-butanediol, 2,3-butanediol, 1,5-pentanediol and the like. Preferred are 1,2-ethanediol, 1,2-propanediol and 1,3-propanediol, and most preferred is 1,2-ethanediol.

As the trivalent alcohol, glycerol and the like can be preferably used.

The above-mentioned ketones are not particularly limited, and normally, one having 3 to 6 carbon atoms is preferably used. Specific examples include acetone, methyl ethyl ketone, methyl butyl ketone, methyl isobutyl ketone and the like. Preferred are acetone and methyl ethyl ketone, and most preferred is acetone.

Nitriles may be cyclic or non-cyclic, saturated or unsaturated, and is not particularly limited. In general, saturated one is preferably used. Normally, nitrile having 2 to 20 carbon atoms, particularly 2 to 12 carbon atoms, especially 2 to 8 carbon atoms, is preferably used. Specific examples include acetonitrile, propionitrile, malononitrile, butyronitrile, isobutyronitrile, succinonitrile, valeronitrile, glutaronitrile, hexanenitrile, heptyl cyanide, octyl cyanide, undecanenitrile, dodecanenitrile, tridecanenitrile, pentadecanenitrile, stearonitrile, chloroacetonitrile, bromoacetonitrile, chloropropionitrile, bromopropionitrile, methoxyacetonitrile, cyanomethyl acetate, ethyl cyanoacetate, tolunitrile, benzonitrile, chlorobenzonitrile, bromobenzonitrile, cyanobenzoic acid, nitrobenzonitrile, anisonitrile, phthalonitrile, bromotolunitrile, methylcyanobenzoate, methoxybenzonitrile, acetylbenzonitrile, naphtonitrile, biphenylcarbonitrile, phenylpropionitrile, phenylbutyronitrile, methylphenylacetonitrile, diphenylacetonitrile, naphthylacetonitrile, nitrophenylacetonitrile, chlorobenzyl cyanide, cyclopropanecarbonitrile, cyclohexanecarbonitrile, cycloheptanecarbonitrile, phenylcyclohexanecarbonitrile, tolylcyclohexanecarbonitrile and the like.

It is preferably acetonitrile, propionitrile, succinonitrile, butyronitrile, isobutyronitrile, valeronitrile, cyanomethyl acetate, ethyl cyanoacetate, benzonitrile, tolunitrile or chloropropionitrile, more preferably acetonitrile, propionitrile, butyronitrile or isobutyronitrile, and most preferably acetonitrile.

Examples of the nitrogen compounds other than nitriles include nitromethane, acetonitrile, triethylamine, pyridine, formamide, N-methylformamide, N,N-dimethylformamide, N,N-dimethylacetamide, N-methylpyrrolidone and the like.

Examples of the sulfur compounds include dimethyl sulfoxide, sulfolane and the like.

Examples of the above-mentioned fatty acids include formic acid, acetic acid, propionic acid and the like. Preferred are formic acid and acetic acid, and most preferred is acetic acid.

The above-mentioned fat and oil may be natural fat and oil from animals and vegetable, synthetic fat and oil, or processed fat and oil. Examples of the vegetable fat and vegetable oil include coconut oil, palm oil, palm kernel oil, flaxseed oil, camellia oil, brown rice germ oil, rape seed oil, rice oil, peanuts oil, corn oil, wheat germ oil, soybean oil, perilla oil, cottonseed oil, sunflower kerel oil, kapok oil, evening primrose oil, shea butter, sal butter, cacao butter, sesame oil, safflower oil, olive oil, avocado oil, poppyseed oil, burdock fruit oil and the like. Examples of the animal fat and animal oil include lard, milk fat, fish oil, beef fat and the like. Furthermore, fat and oil (e.g., hydrogenated oil) obtained by processing the above by fractionation, hydrogenation, transesterification and the like can also be mentioned. Needless to say, medium-chain triglyceride (MCT), partial glycerides of fatty acid and the like can also be used. In addition, a mixture thereof may be used.

The medium-chain triglyceride is not particularly limited and, for example, triglyceride wherein fatty acid has 6 to 12 carbon atoms, preferably 8 to 12 carbon atoms, and the like can be mentioned.

Of the above-mentioned fats and oils, vegetable fat and vegetable oil, synthetic fat and synthetic oil, and processed fat and processed oil are preferable from the aspects of easy handling, odor and the like. Among these, specifically, coconut oil, palm oil, palm kernel oil, rape seed oil, rice oil, soybean oil, cottonseed oil, safflower oil, olive oil, medium-chain triglyceride, partial triglyceride of fatty acid and the like are preferable, and rice oil, soybean oil, rape seed oil, safflower oil, medium-chain triglyceride, partial triglycerides of fatty acid and the like are particularly preferable.

Among the above-mentioned solvents, those acceptable to foods, pharmaceutical products, cosmetics and the like are preferable, and solvents acceptable for foods are more preferable. Since a reaction product can be directly ingested without processing, ethanol, water, fat, oil and a mixture of fat and oil, and a mixed solvent thereof are particularly preferable, and fat, oil and a mixed solvent containing fat or oil are especially preferable. Of fats and oils, medium-chain triglyceride or partial glycerides of fatty acid is preferably used, and medium-chain triglyceride or a mixed solvent containing same is most preferable, from the aspect of solubility of licorice polyphenol and the like.

Needless to say, the above-mentioned solvent may be one contained in a reaction mixture or extraction mixture free of isolation of oxidized coenzyme Q₁₀, which is a starting material. It causes no problem to directly use, as a solvent component for reduction reaction, an extraction solvent used for the extraction of a licorice hydrophobic extract without removal.

For the reduction reaction in the first of the present invention, oxidized coenzyme Q₁₀ and a licorice hydrophobic extract, which are the starting materials, only need to be co-present in the presence of the above-mentioned solvent as necessary. While the method thereof is not limited, in the most preferable embodiment of the first of the present invention, the reduction reaction is performed by extracting a hydrophobic component from licorice or a powder thereof, or a licorice residue, which is obtained by extracting or removing a hydrophilic component of licorice with water or alkali aqueous solution, or the licorice residue after drying, with the above-mentioned organic solvent, fat, oil or a mixture of fat and oil, preferably ethanol and medium-chain triglyceride, evaporating ethanol to give a licorice hydrophobic extract, and adding oxidized coenzyme Q₁₀ obtained by conventionally known methods such as synthesis, fermentation, extraction from naturally occurring substance and the like to a solution of the medium-chain triglyceride in the licorice hydrophobic extract to give a composition containing the oxidized coenzyme Q₁₀ and the licorice hydrophobic extract in coexistence. In addition, in the above-mentioned preferable embodiment, the reduction reaction may be performed by adding other solvents, other reducing agents and other additives as necessary.

While the temperature of reduction reaction in the first of the present invention is not particularly limited, it is preferably performed generally at not less than 20°C, preferably not less than 30°C, and more preferably not less than 40°C.

To exhibit the effect of the present invention at the maximum, the above-mentioned reduction reaction is preferably performed, for example, under a deoxygenation atmosphere. The deoxygenation atmosphere can be formed by substitution with inert gas, reducing pressure, boiling and combining them. At least, replacement by inert gas, that is, use of inert gas atmosphere, is preferable. Examples of the above-mentioned inert gas include nitrogen gas, helium gas, argon gas, hydrogen gas, carbon dioxide gas and the like, preferably nitrogen gas.

In the production method of the first of the present invention, the reduction reaction may be performed in a preparation. In other words, production of reduced coenzyme Q₁₀ by preparing a mixture containing oxidized coenzyme Q₁₀ and a licorice hydrophobic extract, processing the mixture into a preparation form, and reducing oxidized coenzyme Q₁₀ to reduced coenzyme Q₁₀ in the preparation is within the scope of the present invention. The reduction in this case is performed by preservation for a given period or longer, heating or a combination thereof and the like. The given period here is not particularly limited and, for example, not less than 1 day, not less than 1 week, not less than 1 month and the like can be appropriately adopted. In the present invention, while the preparation is not particularly limited, for example, oral administration forms such as capsule (hard capsules, soft capsule, microcapsule), tablet, syrup, liquid preparation, beverage and the like, and external preparation forms such as cream, suppository, toothpaste and the like can be mentioned. The preparation in which the reduction reaction is performed preferably has the above-mentioned oral administration forms, which is more preferably capsule and particularly preferably soft capsule.

Reduced coenzyme Q₁₀ can be conveniently produced by the production method of the present invention. Here, the ratio of reduced coenzyme Q₁₀ to the total amount of coenzyme Q₁₀ at the time of completion of the reaction (i.e., total amount of reduced coenzyme Q₁₀ and oxidized coenzyme Q₁₀) is normally not less than about 20 wt%, preferably not less than about 40 wt%, still more preferably not less than 60 wt%, particularly preferably not less than 80 wt%, especially not less than 90 wt%, most of all not less than 96 wt%.

The reduced coenzyme Q₁₀ obtained by the production method of the first of the present invention can be obtained as crudely purified or pure reduced coenzyme Q₁₀ by appropriately performing, after completion of the reduction reaction, removal of solvent and isolation and purification operation. As mentioned below, the mixed composition after the completion of the reduction reaction can be directly used as a composition containing reduced coenzyme Q₁₀ and a licorice hydrophobic extract, or used after removing the solvent as necessary, followed by formulation into a preparation, in the fields of, for example, pharmaceutical products, foods and the like.

Now the second of the present invention is explained. The second of the present invention includes a stabilization method of reduced coenzyme Q₁₀ by comprising reduced coenzyme Q₁₀ and a licorice hydrophobic extract in coexistence (i.e., stabilization method of reduced coenzyme Q₁₀, comprising forming a composition comprising reduced coenzyme Q₁₀ and a licorice hydrophobic extract in coexistence), and a composition comprising reduced coenzyme Q₁₀ and a licorice hydrophobic extract having glycyrrhizin content of 0.5 wt% or below, wherein the reduced coenzyme Q₁₀ is stabilized by the method.

In the second stabilization method and the stabilized composition of the present invention, reduced coenzyme Q₁₀ to be the object of stabilization may be reduced coenzyme Q₁₀ alone, or a mixture thereof with oxidized coenzyme Q₁₀. When the above-mentioned reduced coenzyme Q₁₀ is a mixture thereof with oxidized coenzyme Q₁₀, the ratio of reduced coenzyme Q₁₀ to the total amount of coenzyme Q₁₀ (i.e., total amount of reduced coenzyme Q₁₀ and oxidized coenzyme Q₁₀) is not particularly limited and, for example, it is not less than 20 wt%, normally not less than 40 wt%, preferably not less than 60 wt%, more preferably not less than 80 wt%, particularly not less than 90 wt%, most of all not less than 96 wt%. While the upper limit is not particularly limited, it is normally not more than 99.9 wt%. Needless to say, when reduced coenzyme Q₁₀ is 100 wt%, reduced coenzyme Q₁₀ may be used alone.

The reduced coenzyme Q₁₀ to be used in the second of the present invention can be obtained, for example, according to conventionally known methods such as synthesis, fermentation, extraction from naturally occurring substance and the like. Preferred are those obtained by reducing oxidized coenzyme Q₁₀ such as existing high pure coenzyme Q₁₀ and the like, or a mixture of oxidized coenzyme Q₁₀ and reduced coenzyme Q₁₀ with a general reducing agent, such as sodium hydrosulfite (sodium dithionite), sodium borohydride, ascorbic acids and the like. More preferred are those obtained by reducing oxidized coenzyme Q₁₀ such as existing high pure coenzyme Q₁₀ and the like, or a mixture of oxidized coenzyme Q₁₀ and reduced coenzyme Q₁₀ with ascorbic acids. Needless to say, reduced coenzyme Q₁₀ obtained by the production method of the first of the present invention can also be used preferably.

Specific examples and detailed explanations of the licorice hydrophobic extract to be used in the stabilization method and stabilized composition of the second of the present invention are the same with those explained in the production method of the first of the present invention.

In the second (stabilization method and composition) of the present invention, the content of the licorice hydrophobic extract to the total composition weight is not particularly limited. However, to sufficiently exhibit the stabilizing effect of reduced coenzyme Q₁₀, it is normally not less than about 1 wt%, preferably not less than about 3 wt%, more preferably not less than about 5 wt%, particularly preferably not less than about 7.5 wt%, based on the dry weight of the extract. While the upper limit is not particularly limited, it is normally not more than about 70 wt%, preferably not more than about 60 wt%, more preferably not more than about 50 wt%, so as to ensure appropriate properties and the like.

In the second (stabilization method and composition) of the present invention, the content of reduced coenzyme Q₁₀ to the total composition weight is not particularly limited. However, to ensure a certain level of the amount of reduced coenzyme Q₁₀ which is the active ingredient of the composition, it is normally not less than about 0.01 wt%, preferably not less than about 0.05 wt%, more preferably not less than about 0.1 wt%, particularly preferably not less than about 0.2 wt%, further preferably not less than about 0.5 wt%. Among these, it is not less than about 1 wt%. While the upper limit is not particularly limited, it is normally not more than 90 wt%, preferably not more than 60 wt%, more preferably not more than 50 wt%, so as to ensure appropriate properties.

In the second (stabilization method and composition) of the present invention, the weight ratio of reduced coenzyme Q₁₀ and licorice hydrophobic extract contained in the composition is not particularly limited. Generally, the weight ratio of reduced coenzyme Q₁₀ to the licorice hydrophobic extract (reduced coenzyme Q₁₀ weight/licorice hydrophobic extract weight) is normally not less than about 1/99, preferably not less than about 5/95, more preferably not less than about 10/90, particularly preferably not less than about 20/80. In the second stabilization method and stabilized composition of the present invention, the stabilization effect can be achieved by using a relatively smaller amount of the licorice hydrophobic extract than that of the licorice hydrophobic extract used in the production method of the first of the present invention. From such aspect, the upper limit of the weight ratio of reduced coenzyme Q₁₀ to the licorice hydrophobic extract (reduced coenzyme Q₁₀ weight/licorice hydrophobic extract weight) is not particularly limited. It is, for example, not more than about 90/1, preferably not more than about 90/10, more preferably not more than about 80/20, particularly preferably not more than about 30/10.

In the second (stabilization method and composition) of the present invention, the reduced coenzyme Q₁₀ and licorice hydrophobic extract need to be contained in coexistence in the composition. Here, "in coexistence" only requests that the both be in contact in some form. The manner of contact is not particularly limited, and the system of the composition may be homogenous or nonhomogenous. In the composition, for example, it may be reaction system wherein reduced coenzyme Q₁₀ and a licorice hydrophobic extract are in contact with each other as a solid, wherein one is present in a liquid layer or dissolved in a solvent and the like, and the other is present as a solid in the liquid layer, wherein reduced coenzyme Q₁₀ is present as a melt, and a licorice hydrophobic extract is present as a solid in the melt, wherein both are present in the liquid layer, forming liquid-liquid two layers, wherein both are present in the same liquid phase and the like. Needless to say, a system showing high contact efficiency between reduced coenzyme Q₁₀ and a licorice hydrophobic extract is effective for stabilizing reduced coenzyme Q₁₀. From this aspect, most preferred is the coexistence of reduced coenzyme Q₁₀ and licorice hydrophobic extract in the same liquid phase.

From the above aspects, in the stabilization method and stabilized composition in the second of the present invention, a solvent is preferably used to allow existence, in the liquid phase, of reduced coenzyme Q₁₀ and/or licorice hydrophobic extract in the composition. The solvent to be used in the second of the present invention is not particularly limited, and examples thereof include organic solvents such as the aforementioned hydrocarbons, fatty acid esters, ethers, alcohols, ketones, fatty acids, nitrogen compounds (including nitriles and amides), sulfur compounds and the like, fats, oils and a mixture of fat and oil, water and the like.

Among the above-mentioned solvents, those acceptable for foods, pharmaceutical products, cosmetics and the like are preferable, and solvents acceptable for foods are particularly preferable. Since a composition containing reduced coenzyme Q₁₀ and licorice hydrophobic extract can be directly ingested, ethanol, water, fat, oil and a mixture of fat and oil are preferable, and fat, oil and a mixture of fat and oil are most preferably used. In addition, since reduced coenzyme Q₁₀ and licorice hydrophobic extract are liposoluble, when water is used as a solvent, a mixture thereof with other solvent capable of dissolving reduced coenzyme Q₁₀ and licorice hydrophobic extract is preferably used. The amount of water to be used is, for example, not more than 90 wt%, preferably not more than 50 wt%, more preferably not more than 30 wt%, to the whole composition.

Other details and preferable examples of the solvents to be used in the second of the present invention are the same as those explained in the first of the present invention.

Needless to say, the above-mentioned solvent may be one contained in a reaction mixture or extraction mixture used as reduced coenzyme Q₁₀. It causes no problem to directly use, as a solvent component for reduction reaction, an extraction solvent used for the extraction of a licorice hydrophobic extract without removal.

In the second of the present invention, the method of preparing a composition containing reduced coenzyme Q₁₀ and a licorice hydrophobic extract in coexistence is not particularly limited. As a method of externally adding reduced coenzyme Q₁₀, for example, reduced coenzyme Q₁₀ and a licorice hydrophobic extract may be simply mixed, or after mixing the both, the above-mentioned solvent may be mixed with the mixture. In addition, a licorice hydrophobic extract may be mixed with a solution containing reduced coenzyme Q₁₀, or reduced coenzyme Q₁₀ may be mixed with a solution containing a licorice hydrophobic extract, or a solution containing reduced coenzyme Q₁₀ and a solution containing a licorice hydrophobic extract may be mixed.

From the aspects of easiness of preparing a composition containing reduced coenzyme Q₁₀ and a licorice hydrophobic extract in coexistence, in one of the most preferable embodiment of the present invention, the preparation is performed by extracting a hydrophobic component from licorice or a powder thereof, or a licorice residue, which is obtained by extracting or removing a hydrophilic component of licorice with water or alkali aqueous solution, or the licorice residue after drying, with the above-mentioned organic solvent or fat, oil or a mixture of fat and oil, preferably ethanol and medium-chain triglyceride, evaporating ethanol to give a licorice hydrophobic extract, and externally adding reduced coenzyme Q₁₀ obtained by conventionally known methods such as synthesis, fermentation, extraction from naturally occurring substance and the like, preferably, reduced coenzyme Q₁₀ obtained by reducing existing highly pure coenzyme Q₁₀, to a solution of the licorice hydrophobic extract in the medium-chain triglyceride to give a composition containing reduced coenzyme Q₁₀ and the licorice hydrophobic extract.

In addition, reduced coenzyme Q₁₀ obtained by the production method of the first of the present invention may be directly utilized, namely, the reaction mixture containing the licorice hydrophobic extract and reduced coenzyme Q₁₀ after completion of the reduction reaction in coexistence may also be directly utilized as the composition of the second of the present invention. Specifically, a composition containing oxidized coenzyme Q₁₀ and a licorice hydrophobic extract is prepared, processed into a preparation form as necessary, and oxidized coenzyme Q₁₀ is reduced in the composition to give reduced coenzyme Q₁₀, whereby a composition containing reduced coenzyme Q₁₀ can be obtained. In the present invention, such embodiment is among other most preferable embodiments.

In the second of the present invention, besides reduced coenzyme Q₁₀, a licorice hydrophobic extract and a solvent, for example, excipient, disintegrant, lubricant, binder, dye, anticoagulant, absorption promoter, solubilizer, stabilizer, flavor, surfactant, active ingredient other than reduced coenzyme Q₁₀, antioxidant and the like can be contained in the composition, and they are not particularly limited.

While the above-mentioned excipient is not particularly limited, for example, sucrose, lactose, glucose, starch, dextrin, mannitol, crystalline cellulose, calcium phosphate, calcium sulfate and the like can be mentioned.

While the above-mentioned disintegrant is not particularly limited, for example, starch, agar, calcium citrate, calcium carbonate, sodium hydrogen carbonate, dextrin, crystalline cellulose, carboxymethylcellulose, tragacanth, alginic acid and the like can be mentioned.

While the above-mentioned lubricant is not particularly limited, for example, talc, magnesium stearate, polyethylene glycol, silica, hydrogenated oil and the like can be mentioned.

While the above-mentioned binder is not particularly limited, for example, ethylcellulose, methylcellulose, hydroxypropylmethylcellulose, hydroxypropylcellulose, tragacanth, shellac, gelatin, pullulan, gum arabic, polyvinylpyrrolidone, polyvinyl alcohol, polyacrylic acid, polymethacrylic acid, sorbitol and the like can be mentioned.

While the above-mentioned dye is not particularly limited, for example, dye such as titanium oxide, food colors, colcothar dye, safflower dye, caramel dye, gardenia dye, tar pigment, chlorophyll and the like can be mentioned.

While the above-mentioned anticoagulant is not particularly limited, for example, stearic acid, talc, light anhydrous silicic acid, hydrated silicon dioxide and the like can be mentioned.

While the above-mentioned absorption promoter is not particularly limited, for example, higher alcohols, higher fatty acids and the like can be mentioned.

While the above-mentioned solubilizing agent for active ingredients is not particularly limited, for example, organic acids such as fumaric acid, succinic acid, malic acid etc., and the like can be mentioned.

While the above-mentioned stabilizer is not particularly limited, for example, benzoic acid, sodium benzoate, ethyl parahydroxybenzoate, beeswax, hydroxypropylmethylcellulose, methylcellulose and the like can be mentioned.

While the above-mentioned flavor is not particularly limited, for example, orange oil, capsicum oil, mustard oil, garlic oil, Caraway oil, clove oil, cinnamon oil, cocoa extract, coffee bean extract, ginger oil, spearmint oil, celery seed oil, thyme oil, onion oil, nutmeg oil, parsley seed oil, peppermint oil, vanilla extract, fennel oil, Pennyroyal oil, peppermint oil, eucalyptus oil, lemon oil, rose oil, rosemary oil, almond oil, ajowan oil, anis oil, amyris oil, angelica route oil, ambrette seed oil, estragon oil, origanum oil, orris root oil, olibanum oil, quassia oil, cascarilla oil, cananga oil, chamomile oil, calamus oil, cardamom oil, carrot seed oil, cubeb oil, cumin oil, grapefruit oil, cinnamon leaf oil, cade oil, pepper oil, costus root oil, cognac oil, copaiba oil, cilantro oil, perilla oil, musk, juniper berry oil, star anise oil, sage oil, savory oil, geranium oil, tangerine oil, Dill oil, neroli oil, tolu balsam oil, basil oil, birch oil, patchouli oil, palmarosa oil, pimento oil, petitgrain oil, bay leaf oil, bergamot oil, Peru balsam oil, benzoin resin, bois de rose oil, hops oil, Boronia Absolute, marjoram oil, mandarin oil, Myrtle oil, Chinese lemon flavor, lime oil, lavandin oil, lavender oil, rue oil, lemongrass oil, lethionine, lovage oil, laurel leaf oil, worm wood oil and the like can be mentioned.

Examples of the above-mentioned surfactant include glycerol fatty acid ester, sucrose fatty acid ester, organic acid monoglyceride, sorbitan fatty acid ester, sorbitan polyoxyethylene fatty acid ester, propylene glycol fatty acid ester, condensed ricinoleic acid glycerides, saponin, phospholipid and the like. Among these, glycerol fatty acid ester, sucrose fatty acid ester, organic acid monoglyceride, sorbitan fatty acid ester, propylene glycol fatty acid ester, condensed ricinoleic acid glycerides and phospholipid can be preferably used.

While the glycerol fatty acid ester is not particularly limited, for example, glycerol having a polymerization degree of 1 - 10 can be mentioned. Examples include glycerol fatty acid esters wherein fatty acid has 6 to 18 carbon atoms and the like. The fatty acid residue constituting glycerol fatty acid ester is not particularly limited, and those wherein fatty acid has 6 to 18 carbon atoms can be preferably used. For example, caprylic acid, capric acid, lauric acid, myristic acid, palmitic acid, stearic acid, isostearic acid, oleic acid, linoleic acid, linolenic acid and the like can be mentioned.

As propylene glycol fatty acid ester, both monoester and diester can be used. While the fatty acid residue constituting propylene glycol fatty acid ester is not particularly limited, those wherein fatty acid has 6 to 18 carbon atoms can be preferably used. For example, caprylic acid, capric acid, lauric acid, myristic acid, palmitic acid, stearic acid, isostearic acid, oleic acid, linoleic acid, linolenic acid and the like can be mentioned.

While the phospholipid is not particularly limited, for example, egg-yolk lecithin, purified soybean lecithin, phosphatidylcholine, phosphatidylethanolamine, phosphatidylserine, sphingomyelin, dicetyl phosphoric acid, stearylamine, phosphatidylglycerol, phosphatidic acid, phosphatidylinositolamine, cardiolipin, ceramide phosphoryl ethanolamine, ceramide phosphoryl glycerol, and a mixture thereof and the like can be mentioned. Needless to say, phospholipid after processing such as hydrogenation, enzymatic degradation and the like can also be used. To improve absorbability of reduced coenzyme Q₁₀, enzyme-degraded phospholipid is preferably used.

While saponin is not particularly limited, sophora saponin, quillaja saponin, purified soybean saponin, yucca saponin and the like can be mentioned.

Examples of the active ingredient other than the above-mentioned reduced coenzyme Q₁₀ include amino acid, vitamin, mineral, polyphenol, organic acid, saccharides, peptide, protein and the like.

Examples of the above-mentioned antioxidant include ascorbic acids, tocopherols, vitamin A, [beta]-carotene, sodium bisulfite, sodium thiosulfate, sodium pyrrosulfite, citric acids and the like. Among these, ascorbic acids and citric acids are particularly preferably used since they preferably suppress oxidation of reduced coenzyme Q₁₀. As ascorbic acids, concentrated fruit juice (extract, powder etc.) of lemon, orange, grapefruit and the like containing ascorbic acids may be used.

The above-mentioned substances may have plural roles. For example, starch may have roles of excipient and disintegrant, and citric acid may have three roles of solubilizer, active ingredient other than reduced coenzyme Q₁₀ and antioxidant.

The composition containing reduced coenzyme Q₁₀ and a licorice hydrophobic extract having glycyrrhizin content of 0.5 wt% or below, which is the second of the present invention, can be used as is, or can also be used by processing into a preparation such as those described in the first of the present invention, namely, oral administration forms such as capsule (hard capsules, soft capsule, microcapsule), tablet, syrup, beverage and the like, external preparation forms such as cream, suppository, toothpaste and the like. Among these, processing into the above-mentioned oral administration form is preferable, processing into a capsule form is particularly preferable, and processing into a soft capsule is especially preferable.

Here, a capsule base is not particularly limited, and gelatin derived from beef bone, cattle skin, pig skin, fish skin and the like, other substrates (e.g., thickening stabilizers such as seaweed-derived products such as carageenan, alginic acid etc., vegetable seed-derived products such as locust bean gum, guar gum etc. and cellulose-containing agents for production, which are usable as food additives) can also be used.

To exhibit the effect of the present invention at the maximum, the stabilization method of the second of the present invention is preferably performed in a deoxygenation atmosphere and the composition of the second of the present invention is preferably prepared and/or preserved under a deoxygenation atmosphere. In addition, the above-mentioned processing into a preparation and preservation after processing are also preferably performed in a deoxygenation atmosphere. The deoxygenation atmosphere can be formed by substitution with inert gas, reducing pressure, boiling and combining them. At least, substitution with inert gas, that is, use of inert gas atmosphere, is preferable. Examples of the above-mentioned inert gas include nitrogen gas, helium gas, argon gas, hydrogen gas, carbon dioxide gas and the like, preferably nitrogen gas.

As mentioned above, in the composition of the second of the present invention containing reduced coenzyme Q₁₀ and a licorice hydrophobic extract, and a processed preparation thereof in an oral administration form and the like, reduced coenzyme Q₁₀ can be preserved stably. The maintenance ratio of reduced coenzyme Q₁₀ after preservation for a predetermined period (e.g., ratio of reduced coenzyme Q₁₀ to coenzyme Q₁₀ after preservation for a predetermined period/ratio of reduced coenzyme Q₁₀ to coenzyme Q₁₀ when preservation was started) is expected to be generally not less than about 80 wt%, preferably not less than about 90 wt%, more preferably not less than about 95 wt%. The above-mentioned predetermined period is, for example, not less than 1 day, preferably not less than 1 week, more preferably not less than 1 month, particularly preferably not less than half year, especially not less than 1 year, most of all not less than 2 years. The temperature during the preservation is not particularly limited as long as it is general condition for preservation of preparations. It is for example 0°C - 60°C, preferably room temperature.

In the composition of the second of the present invention containing reduced coenzyme Q₁₀ and a licorice hydrophobic extract, not only reduced coenzyme Q₁₀, which is an active ingredient, is protected from oxidation and stably maintained, but also a licorice hydrophobic extract having various actions such as visceral fat-lowering effect, metabolic syndrome-preventing and improving effect and the like. Therefore, the effects of both reduced coenzyme Q₁₀ and licorice hydrophobic extract, and synergistic effects thereof can be expected. Thus, the composition is useful as foods such as food with nutrient function claims, food for specified health uses and the like, drinks, pharmaceutical products, animal drugs, pet foods and the like.

### Examples

The present invention is explained in more detail in the following by referring to Production Examples and Examples, which are not to be construed as limitative. The oxidized coenzyme Q₁₀ used in the following Examples was manufactured by Kaneka Corporation (purity 99.5%). In addition, the purity of oxidized coenzyme Q₁₀ and reduced coenzyme Q₁₀, and the ratio of reduced coenzyme Q₁₀ to the total coenzyme Q₁₀ (i.e., reduced coenzyme Q₁₀/(oxidized coenzyme Q₁₀+reduced coenzyme Q₁₀): hereinafter to be also referred to simply as "ratio of reduced coenzyme Q₁₀") were determined by the following HPLC analysis.

### (HPLC analysis conditions)

column: SYMMETRY C18 (manufactured by Waters) 250 mm (length) 4.6 mm (inner diameter), mobile phase; C₂H₅OH:CH₃OH=4:3 (v:v), detection wavelength; 210 nm, flow rate; 1 ml/min, retention time of reduced coenzyme Q₁₀; 9.1 min, retention time of oxidized coenzyme Q₁₀; 13.3 min.

### (Production Example 1) Preparation of licorice hydrophobic extract

Rhizome (1.0 Kg) of licorice (G. glabra) from Afghan was extracted (45°C, 2 hr, twice) with ethanol (5.0 L) and concentrated under reduced pressure to give a concentrated liquid (0.45 L). Then, the concentrated liquid (0.3 L) was concentrated and further treated with activated carbon to give a licorice hydrophobic extract-containing ethanol solution (123.6 g, containing 24.8 g of licorice hydrophobic extract).

### (Production Example 2) Preparation of licorice hydrophobic extract-MCT solution

The licorice hydrophobic extract-containing ethanol solution (63.9 g) of Production Example 1 and medium chain fatty acid triglyceride (hereinafter to be referred to as MCT) (product name; Actor M2; Riken Vitamin Co., Ltd., fatty acid composition C8:C10=99:1, 18.8 g) were mixed, and the mixture was concentrated under reduced pressure to remove ethanol. 28.7 g obtained by concentration under reduced pressure was suction filtered to remove an insoluble material, which was washed with hexane. The resulting recovered liquid was added to the earlier filtrate. MCT (4.5 g) was added to the recovered filtrate (26.2 g) to give a licorice hydrophobic extract-containing MCT solution (30.7 g, containing 8.9 g of licorice hydrophobic extract, glycyrrhizin was undetectable).

### HPLC analysis

### <Preparation of HPLC analysis sample>

The above-mentioned licorice hydrophobic extract-containing MCT solution (1 g) was dissolved in methanol for HPLC, and the total amount was adjusted to 100 ml.

### <HPLC conditions>

column: YMC, J'sphere ODS-H80, 4.6×250 mm
column temperature: 40°C
mobile phase: A=20 mM aqueous phosphoric acid solution B=acetonitrile:methanol (50:50=v/v)
gradient: conditions under which the ratio of B to mobile phase A was maintained constant at 50% for 20 minutes from the start of the analysis, raised at a given rate to reach 80% in 75 minutes after the 20 minutes, maintained constant at 100% from 75 minutes to 80 minutes and maintained constant at 50% from 80 minutes to 100 minutes
flow rate: 1 ml/min
wavelength: UV 282 nm
sample injection volume: 20 µL

### <Analysis results>

The content of each component contained in 1 g of the licorice hydrophobic extract-containing MCT solution was glabrene (4.4 mg), glabridin (30.0 mg), glabrol (6.0 mg), and 4'-O-methylglabridin (5.2 mg).

### <Polyphenol analysis>

As a result of the polyphenol content measurement by Folin-Denis assay and using glabridin (manufactured by Wako Pure Chemical Industries, Ltd.) as a standard substance, the total content of polyphenol in 1 g of the licorice hydrophobic extract-containing MCT solution was 239.1 mg.

### (Production Example 3) Preparation of reduced coenzyme Q₁₀

Oxidized coenzyme Q₁₀ (100 g, manufactured by Kaneka Corporation) and L-ascorbic acid (60 g) were added to ethanol (1000 g), and the mixture was stirred at 78°C to perform a reduction reaction. After 30 hr, the reaction mixture was cooled to 50°C, and ethanol (400 g) was added while maintaining the same temperature. The ethanol solution (containing 100 g of reduced coenzyme Q₁₀) was cooled to 2°C at a cooling rate of 10°C/hr with stirring to give a white slurry. The obtained slurry was filtered under reduced pressure, the wet crystals were washed with cold ethanol, cold water and cold ethanol in this order (temperature of cold solvent used for washing, 2°C) and dried under reduced pressure (20 - 40°C, 1 - 30 mmHg) to give white dry crystals (95 g). All operations except drying under reduced pressure were performed under a nitrogen atmosphere. The ratio of reduced coenzyme Q₁₀ in the obtained crystals was 99.5 wt%.

### (Examples 1 - 2, Comparative Examples 1 - 2)

To a sample bottle (30 mL) were added the licorice hydrophobic extract-MCT solution obtained in Production Example 2, and the crystals of reduced coenzyme Q₁₀ obtained in Production Example 3, in an amount described in Table 1, and they were mixed to give a solution (10 g). The solution was preserved at 40°C in the air, and the residual rates of reduced coenzyme Q₁₀ after preservation for 15 days and 30 days (ratio of reduced coenzyme Q₁₀ after preservation/ratio of reduced coenzyme Q₁₀ when preservation was started) were respectively determined. The results are shown in Table 1. For comparison, the results obtained using only MCT instead of the licorice hydrophobic extract-MCT solution are concurrently shown.

**Table 1**

| | Amount of reduced coenzyme Q₁₀ used | Amount of licorice hydrophobic extract-MCT solution used | Amount of MCT used | reduced coenzyme Q₁₀/licorice polyphenol (weight ratio) | Residual rate of reduced coenzyme Q₁₀ after preservation | |
|---|---|---|---|---|---|---|
| | | | | | 15 days | 30 days |
| Ex. 1 | 0.1 g | 9.9 g | - | 1/23.67 | 93.0% | 85.2% |
| Com. Ex. 1 | 0.1 g | - | 9.9 g | - | 82.1% | 42.1% |
| Ex. 2 | 1.0 g | 9.0 g | - | 1/2.15 | 95.9% | 88.2% |
| Com. Ex. 2 | 1.0 g | - | 9.0 g | - | 91.7% | 76.4% |

From the foregoing results of Examples 1 - 2 and Comparative Examples 1 - 2, it was clarified that a composition containing reduced coenzyme Q₁₀ and a licorice hydrophobic extract in coexistence improves the stability of reduced coenzyme Q₁₀.

### (Comparative Examples 3 - 5)

To a sample bottle (30 mL) were added a turmeric extract (manufactured by MARUZEN PHARMACEUTICALS CO., LTD; polyphenol content 70 wt%) (Comparative Example 3), quercetin (quercetin 2 hydrate, manufactured by Sigma Ltd.) (Comparative Example 4), rutin (rutin hydrate, manufactured by Sigma Ltd.) (Comparative Example 5), reduced coenzyme Q₁₀ crystals obtained in Production Example 3 and MCT, each in an amount described in Table 2, and they were mixed to give a mixed solution (10 g). The mixed solution was preserved at 40°C in the air for 30 days, and the residual rate of reduced coenzyme Q₁₀ after preservation (ratio of reduced coenzyme Q₁₀ after preservation/ratio of reduced coenzyme Q₁₀ when preservation was started) was determined. The results are shown in Table 2.

**Table 2**

| | Amount of reduced coenzyme Q₁₀ used | Plant extract or polyphenol used and amount thereof used | Amount of MCT used | Reduced coenzyme Q₁₀/ polyphenol (weight ratio) | Residual rate of reduced coenzyme Q₁₀ after preservation for 30 days |
|---|---|---|---|---|---|
| Com. Ex. 3 | 0.1 g | turmeric extract 3.4 g | 6.5 g | 1/24 | 57.7% |
| Com. Ex. 4 | 0.1 g | quercetin 2.4 g | 7.5 g | 1/24 | 22.2% |
| Com. Ex. 5 | 0.1 g | rutin 2.4 g | 7.5 g | 1/24 | 1.4% |

From the foregoing results, it was confirmed that licorice hydrophobic extract is particularly superior in the ability to stabilize reduced coenzyme Q₁₀, as compared to other plant extract (turmeric extract) and other polyphenols.

### (Example 3, Comparative Example 6)

To a sample bottle (30 mL) were added the licorice hydrophobic extract - MCT solution obtained in Production Example 2 and oxidized coenzyme Q₁₀ crystals (manufactured by Kaneka Corporation), in an amount described in Table 3, and they were mixed to give a solution (10 g). The solution was preserved at 40°C in the air for 30 days, and the conversion rate of oxidized coenzyme Q₁₀ to reduced coenzyme Q₁₀ after the preservation (ratio of reduced coenzyme Q₁₀ after preservation) was determined. The results are shown in Table 3. For comparison, the results obtained using only MCT instead of the licorice hydrophobic extract-MCT solution are concurrently shown.

**Table 3**

| | Amount of oxidized coenzyme Q₁₀ used | Amount of licorice hydrophobic extract-MCT solution used | Amount of MCT used | reduced coenzyme Q₁₀/licorice polyphenol (weight ratio) | Conversion rate of reduced coenzyme Q₁₀ after preservation for 30 days |
|---|---|---|---|---|---|
| Ex. 3 | 0.1 g | 9.9 g | - | 1/23.67 | 33.7% |
| Com. Ex. 6 | 0.1 g | - | 9.9 g | - | 0.0% |

From the foregoing results of Example 3 and Comparative Example 6, it was clarified that oxidized coenzyme Q₁₀ is converted to reduced coenzyme Q₁₀ when oxidized coenzyme Q₁₀ and a licorice hydrophobic extract are co-present.

### (Example 4)

To a pear-shaped flask (100 mL) were added the licorice hydrophobic extract - MCT solution (19.8 g) obtained in Production Example 2 and oxidized coenzyme Q₁₀ crystals (0.2 g, manufactured by Kaneka Corporation), and the mixture was stirred under a nitrogen atmosphere at 80°C. The conversion rate of oxidized coenzyme Q₁₀ to reduced coenzyme Q₁₀ after lapse of 96 hours (ratio of reduced coenzyme Q₁₀ after preservation) was 98.1 wt%.

### (Comparative Example 7)

To a pear-shaped flask (100 mL) were added rutin (4.8 g, rutin hydrate, manufactured by Sigma Ltd.), MCT (15 g) and oxidized coenzyme Q₁₀ crystals (0.2 g, manufactured by Kaneka Corporation), and the mixture was stirred under a nitrogen atmosphere at 80°C. The conversion rate of oxidized coenzyme Q₁₀ to reduced coenzyme Q₁₀ after lapse of 96 hours (ratio of reduced coenzyme Q₁₀ after preservation) was 48.7 wt%. It was confirmed that rutin is inferior to the licorice hydrophobic extract in the ability to reduce oxidized coenzyme Q₁₀.

### (Formulation Example 1)

To the licorice hydrophobic extract - MCT solution (25 parts by weight) obtained in Production Example 2 were added MCT (50 parts by weight), lysolecithin (5 parts by weight) and beeswax (5 parts by weight) and, after homogenous mixing, reduced coenzyme Q₁₀ crystals (15 parts by weight) obtained in Production Example 3 were added at 40°C. The obtained mixture was processed into a gelatin soft capsule preparation by a conventional method.

### (Formulation Example 2)

To the licorice hydrophobic extract - MCT solution (10 parts by weight) obtained in Production Example 2 were added MCT (60 parts by weight), lecithin (5 parts by weight), beeswax (10 parts by weight) and hydrogenated oil (5 parts by weight) and, after homogenous mixing, reduced coenzyme Q₁₀ crystals (10 parts by weight) obtained in Production Example 3 were added at 40°C. The obtained mixture was processed into a gelatin soft capsule preparation by a conventional method.

### (Formulation Example 3)

To the licorice hydrophobic extract - MCT solution (28 parts by weight) obtained in Production Example 2 were added MCT (56 parts by weight), lysolecithin (5 parts by weight) and beeswax (5 parts by weight) and, after homogenous mixing, oxidized coenzyme Q₁₀ (6 parts by weight) was added at 40°C. The obtained mixture was processed into a gelatin soft capsule preparation by a conventional method. The capsule was preserved at 30°C for 1 month to give a soft capsule preparation wherein oxidized coenzyme Q₁₀ in the capsule was partly reduced to reduced coenzyme Q₁₀.

## Claims

1. A method of producing reduced coenzyme Q₁₀, comprising reducing oxidized coenzyme Q₁₀ to reduced coenzyme Q₁₀ by using a licorice hydrophobic extract.

2. The production method of claim 1, wherein the reduction reaction is performed in the presence of an organic solvent, water, fat, oil or a mixture of fat and oil.

3. The production method of claim 1 or 2, wherein the concentration of the licorice hydrophobic extract in the reaction system at the time of the start of the reaction is not less than 1 wt%.

4. The production method of any one of claims 1 to 3, wherein the concentration of the oxidized coenzyme Q₁₀ in the reaction system at the time of the start of the reaction is not less than 0.1 wt%.

5. The production method of any one of claims 1 to 4, wherein the reduction reaction is performed in a preparation.

6. The production method of claim 5, wherein the preparation is a capsule.

7. The production method of any one of claims 1 to 6, wherein the reduction reaction is performed under a deoxygenation atmosphere.

8. A method of stabilizing reduced coenzyme Q₁₀ against oxidation, comprising forming a composition comprising reduced coenzyme Q₁₀ and a licorice hydrophobic extract in coexistence.

9. A composition comprising reduced coenzyme Q₁₀ and a licorice hydrophobic extract having a glycyrrhizin content of 0.5 wt% or below.

10. The composition of claim 9, wherein the reduced coenzyme Q₁₀ is obtained by reducing oxidized coenzyme Q₁₀ in the composition by using a licorice hydrophobic extract.

11. A composition comprising oxidized coenzyme Q₁₀ and a licorice hydrophobic extract having a glycyrrhizin content of 0.5 wt% or below, which is in an oral administration form.

12. The composition of any one of claims 9 to 11, wherein the content of the licorice hydrophobic extract in the composition is not less than 1 wt%.

13. The composition of any one of claims 9 to 12, wherein the licorice hydrophobic extract comprises licorice polyphenol in an amount of not less than 50 wt%.

14. The composition of any one of claims 9 to 13, which is processed into a preparation form.

15. The composition of claim 14, wherein the preparation form is a capsule.

## Patentansprüche

1. Verfahren zur Herstellung von reduziertem Coenzym Q₁₀, das die Reduzierung von oxidiertem Coenzym Q₁₀ zu reduziertem Coenzym Q₁₀ unter Verwendung eines hydrophoben Lakritzeextrakts umfasst.

2. Herstellungsverfahren gemäss Anspruch 1, wobei die Reduktionsreaktion in Gegenwart von organischem Lösungsmittel, Wasser, Fett, Öl oder einer Mischung aus Fett und Öl durchgeführt wird.

3. Herstellungsverfahren gemäss Anspruch 1 oder 2, wobei die Konzentration des hydrophoben Lakritzeextrakts im Reaktionssystem zu Beginn der Umsetzung nicht weniger als 1 Gew.% beträgt.

4. Herstellungsverfahren gemäss irgendeinem der Ansprüche 1 bis 3, wobei die Konzentration an hydrophobem Coenzym Q₁₀ im Reaktionssystem zu Beginn der Umsetzung nicht weniger als 0,1 Gew.% beträgt.

5. Herstellungsverfahren gemäss irgendeinem der Ansprüche 1 bis 4, wobei die Reduktionsreaktion in einer Zubereitung durchgeführt wird.

6. Herstellungsverfahren gemäss Anspruch 5, wobei die Zubereitung eine Kapsel ist.

7. Herstellungsverfahren gemäss irgendeinem der Ansprüche 1 bis 6, wobei die Reduktionsreaktion unter Deoxygenierungsatmosphere durchgeführt wird.

8. Verfahren zur Stabilisierung von reduziertem Coenzym Q₁₀ gegenüber Oxidation, das die Bildung einer Zusammensetzung umfasst, die nebeneinander reduziertes Coenzym Q₁₀ und einen hydrophoben Lakritzeextrakt umfasst.

9. Zusammensetzung, die reduziertes Coenzym Q₁₀ und einen hydrophoben Lakritzeextrakt mit einem Glycyrrhizingehalt von 0,5 Gew.% oder darunter umfasst.

10. Zusammensetzung gemäss Anspruch 9, wobei das reduzierte Coenzym Q₁₀ durch Reduzieren von oxidiertem Coenzym Q₁₀ in der Zusammensetzung unter Verwendung von hydrophobem Lakritzeextrakt erhalten wird.

11. Zusammensetzung, die oxidiertes Coenzym Q₁₀ und einen hydrophoben Lakritzeextrakt mit einem Glycyrrhizingehalt von 0,5 Gew.% oder darunter umfasst, in einer Form zur oralen Verabreichung.

12. Zusammensetzung gemäss irgendeinem der Ansprüche 9 bis 11, wobei der Gehalt an hydrophobem Lakritzeextrakt in der Zusammensetzung nicht weniger als 1 Gew.% beträgt.

13. Zusammensetzung gemäss irgendeinem der Ansprüche 9 bis 12, wobei der hydrophobe Lakritzeextrakt Lakritzepolyphenol in einer Menge von nicht weniger als 50 Gew.% umfasst.

14. Zusammensetzung gemäss irgendeinem der Ansprüche 9 bis 13, die zu einer Zubereitungsform verarbeitet ist.

15. Zubereitung gemäss Anspruch 14, wobei die Zubereitungsform eine Kapsel ist.

## Revendications

1. Méthode de production de coenzyme Q₁₀ réduite, comprenant la réduction de la coenzyme Q₁₀ oxydée en coenzyme Q₁₀ réduite au moyen d'un extrait hydrophobe de réglisse.

2. Méthode de production selon la revendication 1, dans laquelle la réaction de réduction est réalisée en présence d'un solvant organique, d'eau, d'une matière grasse, d'une huile ou d'un mélange de matière grasse et d'huile.

3. Méthode de production selon la revendication 1 ou 2, dans laquelle la concentration de l'extrait hydrophobe de réglisse dans le système réactionnel au moment du début de la réaction n'est pas inférieure à 1 % en poids.

4. Méthode de production selon l'une quelconque des revendications 1 à 3, dans laquelle la concentration de la coenzyme Q₁₀ oxydée dans le système réactionnel au moment du début de la réaction n'est pas inférieure à 0,1 % en poids.

5. Méthode de production selon l'une quelconque des revendications 1 à 4, dans laquelle la réaction de réduction est réalisée dans une préparation.

6. Méthode de production selon la revendication 5, dans laquelle la préparation est une capsule.

7. Méthode de production selon l'une quelconque des revendications 1 à 6, dans laquelle la réaction de réduction est réalisée sous une atmosphère de désoxygénation.

8. Méthode de stabilisation de la coenzyme Q₁₀ réduite contre l'oxydation, comprenant la formation d'une composition contenant la coenzyme Q₁₀ réduite et un extrait hydrophobe de réglisse en coexistence.

9. Composition contenant la coenzyme Q₁₀ réduite et un extrait hydrophobe de réglisse présentant une teneur en glycyrrhizine inférieure ou égale à 0,5 % en poids.

10. Composition selon la revendication 9, dans laquelle la coenzyme Q₁₀ réduite est obtenue par la réduction de la coenzyme Q₁₀ oxydée dans la composition au moyen d'un extrait hydrophobe de réglisse.

11. Composition contenant la coenzyme Q₁₀ oxydée et un extrait hydrophobe de réglisse présentant une teneur en glycyrrhizine inférieure ou égale à 0,5 % en poids, qui est sous une forme pouvant être administrée par voie orale.

12. Composition selon l'une quelconque des revendications 9 à 11, dans laquelle la teneur de l'extrait hydrophobe de réglisse dans la composition n'est pas inférieure à 1 % en poids.

13. Composition selon l'une quelconque des revendications 9 à 12, dans laquelle l'extrait hydrophobe de réglisse comprend un polyphénol de réglisse en une quantité qui n'est pas inférieure à 50 % poids.

14. Composition selon l'une quelconque des revendications 9 à 13, qui est transformée en une forme de préparation.

15. Composition selon la revendication 14, dans laquelle la forme de préparation est une capsule.
